# FASCICULE DE BREVET EUROPEEN

(11) **EP 3 595 592 B1**
(45) Date de publication et mention de la délivrance du brevet: **03.11.2021**
(21) Numéro de dépôt: 18712977.0
(22) Date de dépôt: 08.03.2018
(51) Int. Cl.: A61F 2/50, C12M 3/00, B33Y 80/00, B29C 64/112

(54) **EQUIPEMENT ET PROCEDE D'IMPRESSION ADDITIVE**
VORRICHTUNG UND VERFAHREN ZUR GENERATIVEN FERTIGUNG
EQUIPMENT AND METHOD FOR ADDITIVE MANUFACTURING

(30) Priorité: 15.03.2017 FR 1752131
(43) Date de publication de la demande: 22.01.2020
(73) Titulaire: Poietis, 33600 Pessac (FR)
(72) Inventeur: GUILLEMOT, Fabien, 33210 Preignac (FR); VIELLEROBE, Bertrand, 33700 Merignac (FR); BOUTER, Jérôme, 33000 Bordeaux (FR); LE BOUFFANT, Evarzeg, 33700 Merignac (FR); VAUCELLE, Romain, 33000 Bordeaux (FR); SOTO, Dan, 33000 Bordeaux (FR)
(74) Mandataire: IP Trust
(86) Numéro de dépôt international: PCT/FR2018/050536
(87) Numéro de publication internationale: WO 2018/167402

(56) Documents cités:
- WO-A1-2016/097619
- WO-A1-2016/097620
- WO-A1-2017/011854

## Description

La présente invention concerne le domaine de l'impression additive tridimensionnelle assistée par laser et plus particulièrement, mais non limitativement, la bioimpression.

L'impression additive tridimensionnelle assistée par laser consiste à projeter des particules vers une cible par un jet produit par la vaporisation locale d'un substrat apportant une énergie cinétique à une particule transférable ou un agrégat de particules contenues dans un fluide porteur.

Les particules transférables peuvent provenir d'un matériau pulvérulent porté par un substrat liquide, ou un matériau transférable liquide.

Elles peuvent être de nature métallique, des composés inorganiques, ou encore des polymères ou des biomatériaux.

Elles peuvent également être constituées par des particules biologiques, par exemple des cellules vivantes.

La présente invention concerne le domaine de la bio-impression réalisée par un procédé de transfert assisté par ordinateur pour la modélisation et l'assemblage de matériaux vivants et non-vivants avec une organisation prescrite en 2D ou en 3D dans le but de produire des structures tridimensionnelles, notamment des structures de bio-ingénierie servant en médecine régénérative, en pharmacologie et pour des études de biologie cellulaire.

L'ingénierie tissulaire vise à concevoir et développer des solutions de substitution biologiquement aptes à remplacer, restaurer ou maintenir les fonctions d'un tissu natif, voire d'un organe. Un exemple est décrit dans l'article Griffith, L. G., & Naughton, G. (2002). Tissue engineering-current challenges and expanding opportunities. Science, 295(5557), 1009-1014.

Pour pallier ces limites, l'impression d'éléments biologiques, plus communément appelée bio-impression, a commencé à être imaginée, comme exposé dans les articles Klebe, R. (1988). Cytoscribing: A Method for Micropositioning Cells and the Construction of Two- and Three-Dimensional Synthetic Tissues. Expérimental Cell Research, 179(2):362-373.

Et Klebe, R., Thomas, C., Grant, G., Grant, A. et Gosh, P. (1994). Cytoscription: Computer controlled micropositioning of cell adhesion proteins and cells. Methods in Cell Science, 16(3):189-192.

### Etat de la technique

On connaît dans l'état de la technique le brevet WO2016097619 décrivant un procédé d'impression d'au moins une encre, ledit procédé comprenant une étape de focalisation d'un faisceau laser de manière à générer une cavité dans un film d'encre, une étape de formation d'au moins une gouttelette d'encre à partir d'une surface libre du film d'encre et une étape de dépôt de ladite gouttelette sur une surface de dépose d'un substrat receveur positionnée à une distance donnée (L) du film, caractérisé en ce que le faisceau laser est orienté à contresens par rapport à la force gravitationnelle (G), la surface libre du film étant orientée vers le haut en direction de la surface de dépose placée au-dessus du film d'encre.

On connaît aussi le brevet WO2014061024 concernant un système pour effectuer un transfert vers l'avant induit par laser (LIFT) sans substrat et/ou à donneur local. Ce système comprend un réservoir comprenant au moins une ouverture. Une source d'énergie configurée de façon à délivrer de l'énergie à un matériau donneur à l'intérieur dudit réservoir. Ce système permet le dépôt de matériau par transfert vers l'avant induit par laser sans aucun besoin d'un substrat donneur. L'invention porte également sur des procédés de transfert vers l'avant induit par laser sans substrat et à donneur local.

### Inconvénients de l'art antérieur

Dans les solutions de l'art antérieur prévoyant une lame revêtue par un fluide contenant les particules à transférer, comme il est nécessaire de remplacer la lame après chaque séquence, cela ne permet pas une bonne maîtrise des caractéristiques du film contenant les éléments transférables, notamment le volume du fluide, l'étalement du fluide sur la surface de la lame, l'homogénéité et l'évolution dans le temps en raison des phénomènes de séchage, d'évaporation, d'évolution des particules vivantes,... Ainsi, l'inconvénient principal des solutions de l'art antérieur concerne à la fois un manque de reproductibilité des impressions et la nécessité d'un recours fréquent à des manipulations, réduisant la productivité.

Dans les solutions prévoyant une cuve ou un réservoir contenant un fluide, les problèmes qui se posent concernent la maîtrise de la génération du jet à l'interface air-liquide, car les matériaux à transférer ont tendance à sédimenter et à se retrouver ainsi loin de la cible. Ces solutions ne permettent pas de ce fait de transférer dans des conditions reproductibles les particules contenues dans un fluide. Il s'agit d'un inconvénient générique connu également pour les solutions de bio extrusion ou de jet d'encre.

De façon générale, les solutions de l'art antérieur ne sont pas adaptées à un processus industriel d'impression de milieux liquides contenant des particules, en raison de la difficulté à cibler précisément les particules contenues dans le fluide et de la nécessité de changer régulièrement le support. Ces changements de support impose des manipulations se traduisant par le manque de reproductibilité constaté.

Un problème secondaire que l'invention vise à remédier par certaines de ses variantes concerne l'abandon de l'interaction du laser avec une couche sacrificielle, par exemple un revêtement d'or.

Pour les solutions de l'art antérieur mettant en œuvre une couche sacrificielle, le jet formé par un laser excitant cette couche provoque le transfert de matière en provenance de cette couche pouvant entraînant des problèmes de toxicité, de projection de particules autre que les particules à transférer.

Elles impliquent également la destruction locale du substrat lors de chaque tir, ce qui créé des inhomogénéités et impose un changement répété du substrat.

### Solution apportée par l'invention

On entend par inhomogénéité du film de bioencre au sens du présent brevet toute zone du film ayant des caractéristiques locales propres en terme de composition : particules, espèces biochimiques (facteur de croissance, molécules, ions), biomatériaux.

Les termes «zone inhomogène », « variations locales de composition », «zone de composition spécifique » ont la même signification technique au sens du présent brevet.

La solution consiste à rendre homogène en épaisseur et en densité volumique les inhomogénéités du film de fluide positionné dans la zone d'interaction laser lors de l'impression additive tridimensionnelle assistée par laser. Elle consiste également à permettre le remplissage de façon répétée et maîtrisée par le fluide ladite zone de d'interaction. Une telle solution nécessite par contre la mise en place d'un procédé d'impression par laser n'utilisant pas de couche sacrificielle pour la génération des jets de matière ce qui implique de fait une interaction laser - matière qui a lieu directement dans le fluide. Ainsi, le procédé de génération de la bulle de cavitation puis du jet de matière sera totalement différent de l'art antérieur général.

Les avantages de cette solution sont nombreux :
- elle permet d'apporter de façon contrôlée et reproductible le fluide inhomogène vers la zone d'interaction en évitant toute manipulation (pipettage, étalement, nettoyage...). Elle rend donc le processus plus sécurisant et plus fiable.
- elle permet de moduler la composition de l'encre en mixant plusieurs liquides (espèces chimiques, biomatériaux liquides,...) et plusieurs types de particules (cellules, biomatériaux,...) dans un même type d'encre.
- elle permet l'utilisation d'un système fluidique pilotable qui recharge de façon continue ou pseudo-continue la zone d'interaction permettant ainsi de gagner en productivité sur l'impression.
- elle permet d'obtenir un film de fluide homogène en épaisseur sur la surface de la zone d'interaction ce qui pour but de rendre l'impression beaucoup plus reproductible et homogène au niveau des gouttelettes imprimées sur la surface réceptrice.
- Optionnellement, les films peuvent avoir une pente ou une forme appropriée à la fois à la géométrie des entrées / sorties et au mode de remplissage (continu, discontinu, par aller- retour, etc...)
- elle permet l'ajustement de l'épaisseur du film grâce à des jeux de paramètres optimisés (débit, section, forme...) du système fluidique. Ainsi, la hauteur des jets de matière peut être adaptée par ce biais, ce qui peut être très intéressant pour imprimer sur des surfaces non planes.

Par ailleurs, l'invention permet l'utilisation de moyens d'imagerie corrélés aux tirs lasers afin de cibler les inhomogénéités dans le fluide de façon maîtrisée. Pour ce faire, la zone d'interaction doit reposer sur une matière transparente à la fois pour le laser et pour des moyens d'acquisition d'image.

Même si cette solution est compatible d'une impression laser basée sur l'utilisation d'une couche sacrificielle (typiquement une couche métallique d'or ou d'argent), elle est préférentiellement destinée à l'impression par laser sans recours à une couche sacrificielle. Une telle solution doit donc assurer la création de jets reproductibles et répétables dans le champ lors d'une interaction directe entre le laser et le fluide contenant les inhomogénéités. Pour ce faire, un certain nombre de paramètres d'impression, listés ci-après, sont nécessaires car la génération des jets est très difficile à obtenir dans cette condition d'impression sans couche sacrificielle :
- le laser émet des impulsions courtes en régime picoseconde ou femtoseconde avec un niveau d'énergie compris entre 1 à 40 microjoules, et de préférence de 5 et 20 microjoules afin d'optimiser la génération du plasma laser dans le fluide. A la lecture des exemples de réalisation qui seront décrits dans ce document, la preuve de ces performances sera apportée.
- le laser émet des d'impulsions laser en régime nanoseconde avec une énergie d'énergie de 0,5 à 20 milli joules pour permettre la génération de bulle puis de jets sans couche sacrificielle
- le laser émet préférentiellement des impulsions dans le domaine proche IR pour éviter tout effet ioinisant sur les cellules tout en étant suffisamment absorbable par le milieu. Pour optimiser ce dernier paramètre, il serait tout à fait envisageable d'utiliser un laser dans l'UV ou le moyen IR, voir même dans le visible afin de maximiser le taux d'absorption par le milieu.
- des mesures de propriétés caractéristiques du fluide présent dans la zone d'interaction sont réalisées (densité, viscosité, épaisseur du film, etc.) afin de moduler ou d'optimiser les paramètres lasers et de rendre l'impression la plus homogène possible.
- les images des inhomogénéités dans le fluide permettent de viser des zones spécifiques (nombre ou type de particules), ce qui là encore permet de rendre l'impression homogène et surtout conforme au fichier d'impression numérique puisque le nombre de inhomogénéités imprimées peut être directement contrôlé par le biais de ces moyens d'imagerie ou plus généralement de caractérisation piloté par un calculateur pour l'interprétation des données acquises.

Dans un tel contexte, il n'y a plus de problématique liée à l'impression de débris provenant de la couche sacrificielle vers le substrat d'impression, assurant ainsi une viabilité plus élevée des cellules dans le cadre de la bio-impression.

### Solution apportée par l'invention

L'invention concerne selon son acception la plus générale un équipement d'impression additive comportant un moyen d'excitation énergétique orientable pour produire une interaction ponctuelle avec un fluide recouvrant une lame, afin de provoquer un jet orienté en direction d'une cible, ledit fluide étant constitué par un vecteur liquide contenant des particules transférables ou par un biomatériau liquide transférable, caractérisé en ce que :
- ledit fluide forme un film liquide d'une épaisseur inférieure à 500 *µ*m,
- sur une lame présentant au moins une zone permettant l'interaction avec le laser dans laquelle débouche au moins une entrée, ladite zone d'interaction débouchant dans au moins une sortie, ladite zone d'interaction présentant une ouverture dont la section est au moins 3 fois supérieure à la taille médiane des inhomogénéités présentes dans le fluide
- l'équipement comportant en outre des moyens de circulation du fluide entre ladite entrée (7) et ladite sortie.

Cette lame définit une zone avec un fond de préférence plat, positionné pour permettre l'interaction avec le faisceau d'excitation énergétique, cette zone étant entourée par une bordure présentant un orifice d'entrée et un orifice de sortie, pour assurer la présence dans cette zone d'interaction avec le moyen d'excitation énergétique d'un film qui peut être transitoirement statique, déposé sur la zone, et à d'autre moment formée par une circulation du fluide assurant le renouvellement des particules transférables et le déplacement par rapport à l'axe d'excitation énergétique.

Dans les solutions « statiques », il est nécessaire de remplacer la lame après chaque séquence. Cela ne permet pas une bonne maîtrise des caractéristiques du film contenant les éléments transférables, notamment le volume du fluide, l'étalement du fluide sur la surface de la lame, l'homogénéité et l'évolution dans le temps en raison des phénomènes de séchage, d'évaporation, d'évolution des particules vivantes,...

Les lames de l'art antérieur comportant un revêtement statique d'une encre, nécessitant le changement de la lame après chaque utilisation, ce qui ne permet pas d'optimiser l'utilisation des éléments transférables.

Selon des variantes particulières de mise en œuvre de l'équipement selon l'invention,
- l'épaisseur dudit film est comprise entre 50 et 100 *µ*m
- l'épaisseur dudit film est comprise entre 5 et 10 fois la taille nominale desdites particules transférables
- la surface de ladite zone d'interaction est supérieure à 0,05 mm²
- ladite entrée débouche dans une partie latérale de ladite zone d'interaction
- ladite zone d'interaction présente une partie périphérique débouchant latéralement dans ladite sortie
- ladite entrée et ladite sortie sont constituées par des canaux tubulaires raccordés à la zone de raccordement, l'axe longitudinal desdits canaux tubulaires formant avec le plan transversal de la zone d'interaction compris entre 15° et 35°
- lesdits moyens de circulation du fluide entre ladite entrée et ladite sortie comprennent des moyens de pilotage du débit d'injection (ou de la pression positive exercée sur le fluide) et du débit d'aspiration pour contrôler le débit du fluide (ou de la pression négative exercée sur le fluide) dans la zone d'interaction
- lesdits moyens de pilotage du débit d'injection et du débit d'aspiration sont asservis à la mesure de l'épaisseur du film, pour contrôler l'épaisseur du film dans la zone d'interaction
- l'équipement comporte une pluralité de zones d'interaction comportant chacune une entrée et une sortie
- deux au moins desdites zones d'interaction présentent des entrées et/ou des sorties communes
- ledit moyen d'excitation énergétique est constitué par un laser
- la zone d'interaction est transparente dans la bande de longueur d'onde du laser et de l'imagerie et ne présente pas de couche sacrificielle
- ledit fluide est chargé d'un pigment absorbant dans la longueur d'onde d'émission du laser
- l'équipement comporte des moyens d'imagerie de la zone d'interaction pour le pilotage du laser en fonction de la densité de particules
- ledit laser émet des impulsions en régime picoseconde ou femtoseconde avec un niveau d'énergie compris entre 5 et 20 microjoules, le niveau d'énergie par impulsion étant commandé par un calculateur en fonction du résultat de mesure de caractéristiques du fluide présent dans la zone d'interaction, lesdites mesures comprenant la densité en particules, et/ou la viscosité, et/ou l'épaisseur du film
- ledit moyen d'excitation énergétique est constitué par un générateur d'ondes acoustiques
- l'équipement comporte un moyen d'imagerie de la zone d'interaction et de sélection du type de particule à transférer.

L'invention concerne aussi un procédé d'impression additive par un équipement comportant un moyen d'excitation énergétique orientable pour produire une interaction ponctuelle avec un fluide recouvrant une lame, afin de provoquer un jet orienté en direction d'une cible, ledit fluide étant constitué par un vecteur liquide contenant des inhomogénéités (particules, ou biomatériaux ou espèces chimiques) transférables ou par un biomatériau liquide transférable, caractérisé en ce que ledit fluide forme un film liquide d'une épaisseur inférieure à 500 *µ*m circulant entre un conduit d'entrée (7) et un conduit de sortie d'une lame présentant au moins une zone permettant l'interaction avec le laser, et dans laquelle débouche au moins une entrée (7).

Selon une variante particulière, le niveau d'énergie par impulsion est commandé par un calculateur en fonction du résultat de mesure de caractéristiques du fluide présent dans la zone d'interaction, lesdites mesures comprenant la densité en particules, et/ou la viscosité, et/ou l'épaisseur du film.

### Description détaillée d'un exemple non limitatif de l'invention

La présente invention sera mieux comprise à la lecture de la description détaillée d'un exemple non limitatif de l'invention qui suit, se référant aux dessins annexés où :
- la figure 1 représente une vue schématique en coupe d'un équipement selon l'invention
- la figure 2 représente une vue schématique d'un équipement selon l'invention avec le système optique.
- la figure 3 représente une vue schématique de dessus d'une lame pour un équipement selon l'invention
- la figure 4 représente une vue 3D de différentes variantes de l'équipement selon l'invention implémentées expérimentalement
- la figure 5 représente une vue 3D de différentes variantes de l'équipement selon l'invention comportant une rainure permettant de faire l'image du film d'encre par la tranche
- la figure 6 représente une vue 3D d'une variante de l'équipement selon l'invention ainsi que des images de films d'encre obtenues dans cette configuration
- la figure 7 représente une vue 3D d'une variante multi-cavités de l'équipement selon l'invention
- la figure 8 représente une série de schémas explicatifs du processus d'interaction laser-matière sans couche sacrificielle mis en œuvre dans l'équipement selon l'invention
- la figure 9 représente une série de photographies montrant le jet de matière généré par interaction laser - matière sans couche sacrificielle au sein de l'équipement selon l'invention
- la figure 10 représente une image prise au microscope d'un champ imprimé de gouttelettes d'eau au sein de l'équipement selon l'invention en mode statique (sans rechargement continu)
- la figure 11 représente une image prise au microscope d'un champ imprimé de gouttelettes contenant des microbilles au sein de l'équipement selon l'invention en mode statique (sans rechargement continu)
- la figure 12 représente une image prise au microscope d'un champ imprimé de gouttelettes contenant des cellules au sein de l'équipement selon l'invention en mode statique (sans rechargement continu)
- la figure 13 représente une image prise au microscope d'un champ imprimé de gouttelettes d'eau réalisé au sein de l'équipement selon l'invention en mode dynamique (avec rechargement continu)
- la figure 14 représente une image prise au microscope d'un champ imprimé de gouttelettes contenant des cellules ainsi que de la mélanine (absorbeur) au sein de l'équipement selon l'invention en mode statique (sans rechargement continu)
- les figures 15 à 17 représentent des vues d'un autre exemple de réalisation d'une lame selon l'invention.

### Description d'une première variante d'un équipement selon l'invention

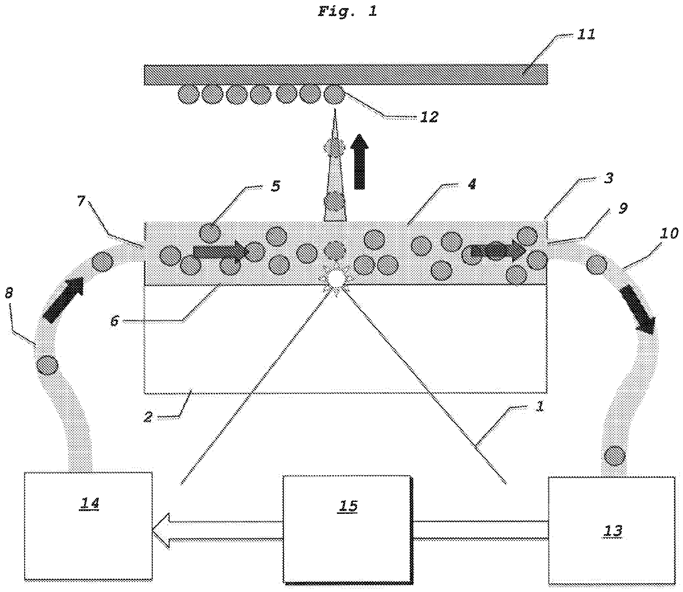

La figure 1 représente une première variante de réalisation d'un équipement, mettant en œuvre une excitation par un faisceau laser focalisé générant une impulsion (1).

Une lame (2) en verre ou en matière transparente définit une cavité (3) dans laquelle circule un fluide porteur (4) contenant des particules transférables (5).

La profondeur de cette cavité est inférieure à 500 *µ*m et préférentiellement de 50 à 100*µ*m d'épaisseur, ainsi on évite les phénomènes de sédimentation dans la cavité (3).

Cette cavité (3) est formée par moulage, usinage, soufflage (verre) ou encore par impression 3D (FDM, SLS, SLA, DLP, DMLS, EBM,CLIP, MultiJet,...) et présente une section circulaire, ou rectangulaire, ou ovale, ou d'autres formes géométriques. Sa surface transversale (6) définit une zone de travail qui peut être balayée par le faisceau laser (1) et visualisée sur un capteur via un rétro-faisceau optique.

Le fluide porteur (4), poussé par un système de pompage (15), pénètre dans la cavité (3) par une ouverture d'entrée (7) raccordée à un conduit d'alimentation (8) lui même raccordé à un réservoir d'alimentation (14), et est évacué via un orifice de sortie (9) vers un conduit (10) d'évacuation et/ou d'aspiration.

Le conduit 'évacuation et/ou d'aspiration (10) débouche dans un réservoir de récupération (13) contenant le fluide porteur (4) chargé avec des particules transférables (5). Une pompe (15) assure la circulation du fluide porteur (4) chargé avec des particules transférables (5). Le réservoir d'alimentation (14) et le réservoir de récupération (13) peuvent être séparés ou bien ne former qu'un seul réservoir dans le cas où on désire faire recirculer le même fluide dans le système plusieurs fois. Dans cette configuration, l'intérêt est de maximiser le nombre de particules imprimées dans le fluide circulant.

Optionnellement, le système comprend plusieurs jeux de réservoirs (14 et 13), contenant chacun un fluide porteur chargé d'inhomogénéités de natures différentes. Une vanne permet de sélectionner l'un des réservoirs, pour permettre la dépose de particules de natures différentes et la formation de couches différenciées sur la cible (11).

Le débit de fluide porteur (4) est ajusté pour assurer le déplacement de la zone de travail des particules transférables (5) à une vitesse permettant de sélection par des moyens appropriés (imagerie, spectroscopie,...) et activer celles qui sont sélectionnées par un tir laser.

La cible (11) est mobile dans un plan X, Y parallèle au fond (6) de la cavité (3) pour déterminer le point de dépose de la particule transférée (12) et éventuellement selon une direction perpendiculaire, pour ajuster la distance parcourue par la particule (5) à transférer. Dans ce cas, il est possible de moduler la taille des gouttelettes déposées sur le substrat receveur d'impression.

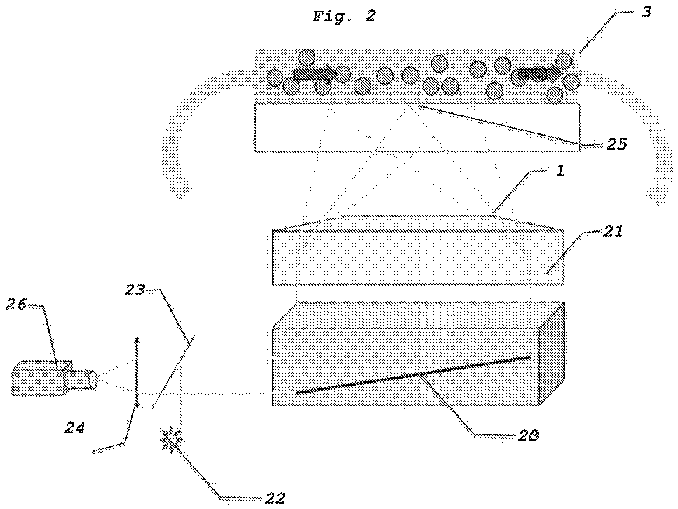

La figure 2 représente une vue en coupe du dispositif complété avec le système optique.

Ce système optique est constitué par deux miroirs oscillants angulairement (de type galvanomètres) (20), permettant de balayer la zone de tirs laser, et par un premier bloc optique (21) constitué d'une lentille de balayage, de type lentille F-Theta, permettant de former un spot laser dont le diamètre sur le plan de travail est le plus petit et le plus constant possible. Ce premier bloc optique (21) est constitué de manière connue par un système de plusieurs lentilles.

En amont des miroirs de balayage (20), le système optique comprend une source laser (22) dont le faisceau est renvoyé vers les miroirs de balayage (20) par un miroir dichroïque (23).

Un deuxième bloc optique (24) forme une image de la zone de travail (25) par le rétro-faisceau traversant le miroir dichroïque (23), sur un capteur (26).

### Description d'une seconde variante d'un équipement selon l'invention

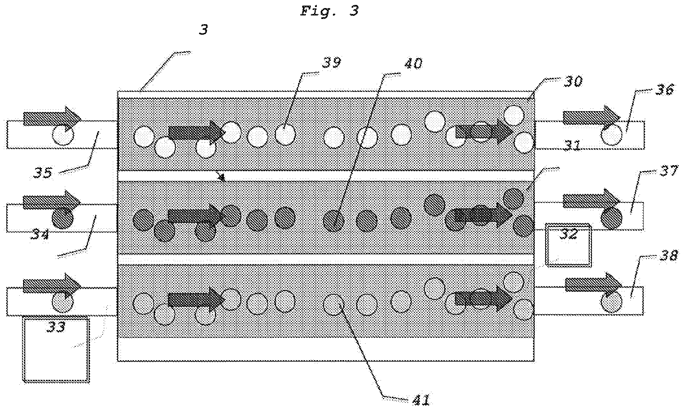

La figure 3 représente une vue de dessus d'une variante d'une lame (2) selon l'invention.

Elle comprend trois circuits formés de trois cavités (30, 31, 32) parallèles, s'étendant chacune entre un conduit d'alimentation (respectivement 33 à 35) et un conduit d'évacuation respectivement (36 à 38).

Chaque circuit assure la circulation d'un fluide porteur contenant des inhomogénéités transférables respectivement (39 à 41) de natures potentiellement différentes. En effet, soit elles sont de même nature ce qui pourrait permettre d'imprimer sur des champs plus importants en taille ou bien d'accélérer le processus d'impression (gain de productivité), soit elles sont de nature différente ce qui pourrait permettre de fabriquer des objets complexes et personnalisés (gain sur l'éventail des objets fabricables), c'est le côté « multicouleur » apporté par ce genre d'architecture.

Pour sélectionner l'une des cavités, la lame (2) peut être déplacée mécaniquement selon une direction perpendiculaire à l'axe principal des trois cavités, ou bien le balayage du faisceau laser permet de couvrir l'entièreté de la lame (2).

### Description de mises en œuvre de différents équipements selon l'invention

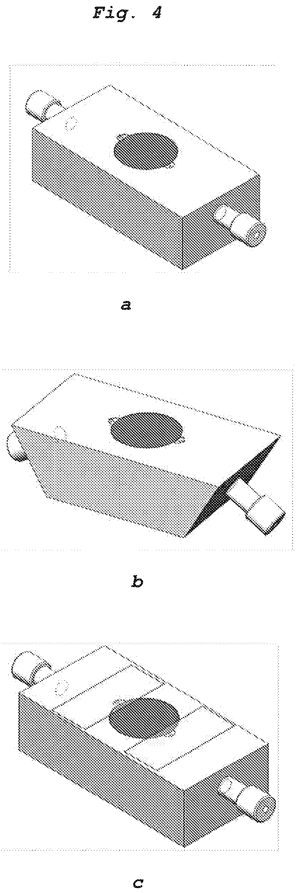

La figure 4 représente une vue de 3 architectures possibles de l'équipement. La figure 4.a représente une vue où les conduits d'alimentation (8) et d'évacuation (10) sont parallèles à la lame (2) alors que la figure 4.b illustre une situation où les conduits arrivent avec un angle par rapport à la lame qui peut être compris entre 0 et 90°. L'intérêt de l'une ou l'autre des solutions réside dans la capacité à gérer les débits, les volumes morts ou encore les angles afin d'éviter tout problème de bouchage ou de continuité dans l'écoulement du fluide (4) et à garantir dans le même temps l'obtention d'un film de fluide homogène dans la cavité ouverte (3) (partie circulaire sur les figures où sont focalisés les tirs lasers). La figure 4.c est une variante des précédentes solutions où des lamelles en verre ont été placées au niveau des entrées (7) et sorties (9) du fluide (4) pour orienter / guider son écoulement sous la forme d'un film homogène dans la cavité (3). Les architectures illustrées ici ne sont pas exhaustives. En effet, la forme, le positionnement et l'angle des conduits, la taille et la forme de la cavité et de la lame, les matériaux utilisés, la forme et le positionnement des orifices d'entrée (7) et de sortie peuvent différer des exemples illustrés ici.

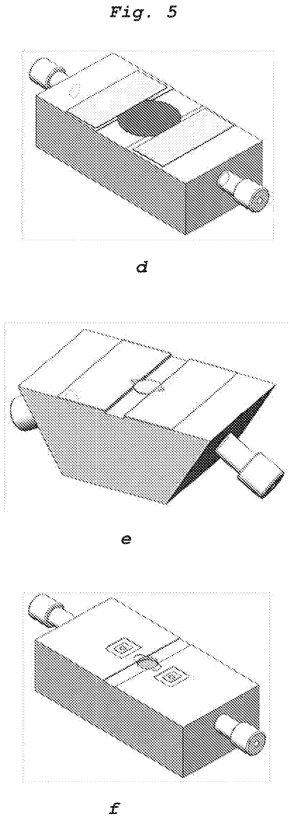

La figure 5 représente une vue de 3 architectures possibles de l'équipement proches de celles présentées dans la figure 4. Elles ont toutes pour point commun de présenter dans cette nouvelle configuration une rainure sur la partie haute dont la hauteur correspond à celle de la cavité (3) et dont le positionnement intersecte la zone ouverte de la cavité (3). L'intérêt d'une telle ouverture est de permettre l'observation du film par des moyens de visualisation (imagerie) placés de façon perpendiculaire à l'équipement. Ainsi, il est possible de suivre l'évolution du film dans le temps, ce qui permet d'adapter au mieux l'énergie des tirs lasers à l'épaisseur réelle du film au cours de l'impression ou inversement adapter l'épaisseur du film à l'énergie du tir laser. Là encore, les architectures illustrées ici ne sont pas exhaustives. A nouveau, la forme, le positionnement et l'angle des conduits, la taille et la forme de la cavité et de la lame, les matériaux utilisés, la forme et le positionnement des orifices d'entrée (7) et de sortie, la forme, la taille et le positionnement des rainures peuvent différer des exemples illustrés ici.

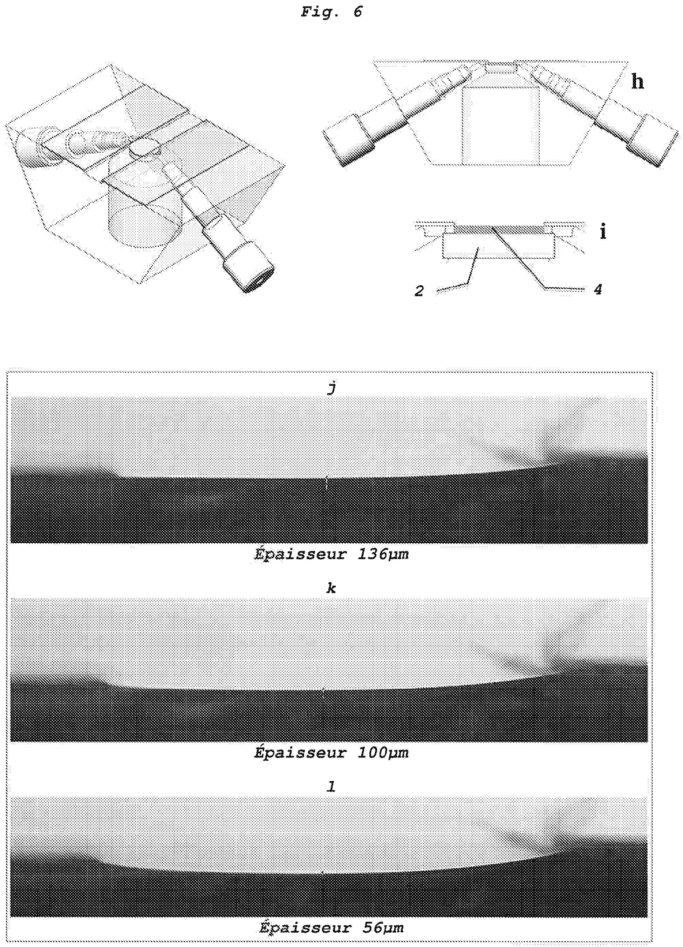

La figure 6 présente des résultats obtenus sur l'une des variantes de l'équipement selon l'invention, initialement illustrée sur la figure 5.e. Sur la figure 6.h, une vue par transparence permet de mettre en lumière l'architecture interne de l'équipement dans cette configuration. Les conduits d'alimentation et d'évacuation arrivent avec un angle par rapport à la cavité (3) et à la lame (2). Le lien entre les conduits et la zone de la cavité se fait par des tuyaux de petit diamètre pour être compatible avec les épaisseurs de film recherchées inférieures à 500*µ*m. Pour autant, il ne faut pas que la section de ces conduits soit trop petite afin d'éviter tout phénomène comparable à ceux constatés dans les systèmes à buses (orifices) qui se bouchent facilement et qui apportent un stress mécanique important aux cellules, impactant alors leur viabilité dans le temps. C'est pour cette raison que les conduits auront un diamètre de préférence supérieur à 200*µ*m, soit au moins 10 fois la taille moyenne des cellules ou des particules imprimables par cet équipement. On peut remarquer que l'équipement est percé en son centre par un trou (cylindre) permettant au laser d'être focalisé dans la cavité en passant à travers la lame (2), transparente à sa longueur d'onde. Sur la figure 6.i, une vue zoomée de la cavité (3) se trouvant au dessus de la lame (2) sur laquelle est envoyé le fluide (4) permet de montrer que les orifices d'entrée (7) et de sortie respectivement (7 et 9) sont constitués pour partie par les conduits d'alimentation et d'évacuation respectivement(8 et 10) et par les lamelles de verre placées sur le dessus de l'équipement. L'association des conduits et des lamelles permet de diriger proprement le fluide (4) sur la lame (2). Des images prouvant qu'il est possible d'obtenir des films minces sont représentées dans les photographies 6.j, 6.k et 6.l obtenues par imagerie (caméra et objectif de reprise d'image) de façon transversale à l'équipement. En fonction des paramètres de pression du fluide (4) en entrée (7) et d'aspiration du fluide (4) en sortie, il est possible de moduler l'épaisseur centrale du film. A titre d'exemple, des épaisseurs de 136*µ*m, 100*µ*m et 56*µ*m ont été obtenues expérimentalement et sont illustrées sur les figures 6.j, 6.k et 6.l.

Le monitoring de ces épaisseurs corrélé à l'adaptation des paramètres d'excitation laser (énergie, focalisation, etc.) permet un ajustement fin des jets générés par l'absorption du laser. La forme supérieure du film n'est pas forcément plane comme on peut le constater sur les photographies. Celle-ci dépend des paramètres du fluide (4) (viscosité, densité, débit...) et des paramètres de pression / aspiration dudit fluide. La zone des tirs laser peut être adaptée spatialement à une partie du film où l'épaisseur est constante. Ladite zone peut aussi correspondre à la totalité de la cavité mais dans ce cas, les paramètres lasers seront adaptés aux variations d'épaisseur du film dans le champ visé.

La caractérisation du film pourrait également être effectuée par d'autres moyens que l'imagerie citée ici, on peut par exemple penser à des moyens d'analyse spectroscopique, de mesure de distance, d'ombroscopie selon une ligne, etc.

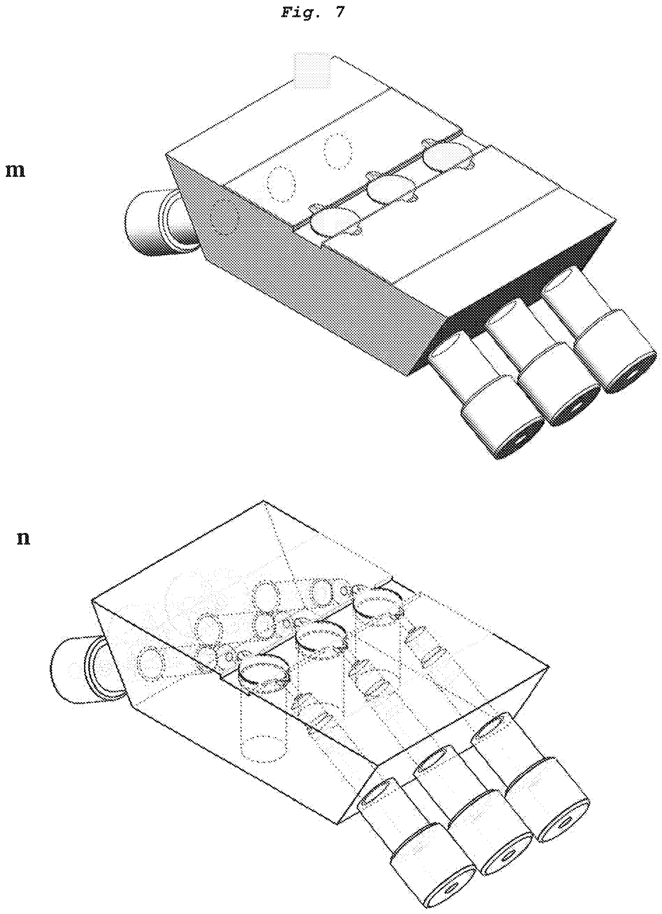

La figure 7 représente une version 3D de la solution présentée sur la figure 3. Cette réalisation, montrée en 3D sur la figure 7.m, comporte 3 cavités alimentées chacune par des conduits d'alimentation et d'évacuation dédiés. Une rainure est présente sur le dessus pour permettre l'observation des films d'encre, au moins ceux placés aux extrémités de l'équipement. Par ailleurs, une vue par transparence est proposée sur la figure 7.n. Elle permet de voir les trois perçages permettant au laser de passer à travers la pièce jusqu'à chaque cavité (3) équipée d'une lame (2). Il est évident que cet exemple est purement illustratif des larges possibilités de designs qui sont envisageables pour cet équipement. En effet, on pourrait l'imaginer avec :
- 2 cavités ou plus de 3 cavités
- un seul conduit d'alimentation et / ou un seul conduit d'évacuation communs à toutes les cavités
- une forme de cavité différente (carrée, canal, ovale, losange, etc.)
- des conduits arrivant parallèlement ou perpendiculairement à la surface des lames

Les exemples cités ici ne sont donc pas limitatifs des architectures que l'équipement selon l'invention pourrait avoir.

### Description détaillée du procédé d'impression sans couche sacrificielle selon l'invention

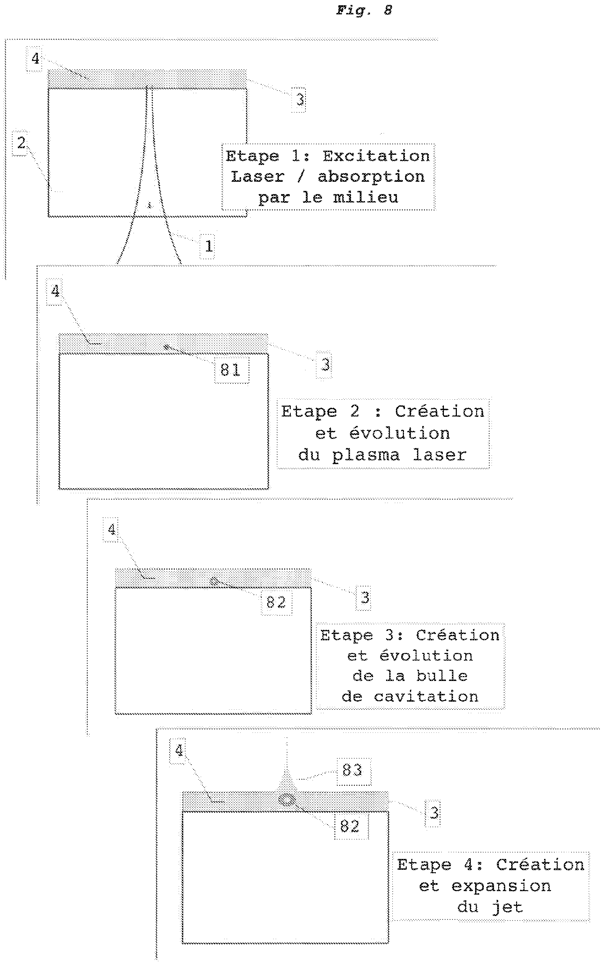

La figure 8 décrit les principales étapes de l'interaction entre le laser et la matière dans le cadre de l'impression de liquides homogènes ou contenant des inhomogénéités.
- La première étape consiste à focaliser le laser sur la matière, ici l'encre disposée sous la forme d'un film (4) dans la cavité (3). La façon de focaliser le laser impacte directement le volume qui va absorber l'énergie déposée. On parle dans ce cas de fluence laser (énergie rapportée à la surface voir au volume). Comme le processus n'utilise pas de couche sacrificielle, c'est l'encre et en majeure partie son milieu liquide qui absorbe l'énergie du laser. De fait, le choix de la longueur d'onde du laser et de son énergie a une incidence directe sur la capacité d'absorption du film (4). Dans le cas de la bio-impression, le milieu est essentiellement constitué d'eau qui a des pics d'absorption bien connus au niveau spectral. On pourra donc chercher à maximiser cette absorption en choisissant des sources lasers correspondant à ces maximas (raies d'absorption de l'eau dans l'infrarouge par exemple). On pourra également chercher à maximiser l'absorption par le biais d'absorbeurs placés dans l'encre (molécules, colorants, particules). Dans les exemples illustrés dans cette présente invention, le laser utilisé travaille à 1030 nm de longueur d'onde (ytterbium) pour une durée d'impulsion couvrant une zone allant de 10 picosecondes à 400 femtosecondes et des énergies entre 1 et 40 *µ*Joules. De préférence, nous avons utilisé le laser avec une durée d'impulsion de 10 picosecondes pour une énergie par impulsion de 10 à 14 *µ*Joules.
- La seconde étape correspond à la création du plasma (81) qui est le résultat de la dissociation de la matière suite à l'absorption du laser par le fluide du film (4). Ce plasma est constitué d'un mélange d'atomes, d'ions, d'électrons, de résidus moléculaires....

Il est créé sur des temps extrêmement courts, typiquement quelques picosecondes après l'absorption laser et il a un temps de « vie » lui aussi très court de l'ordre de la microseconde. La taille du plasma (81), sa dynamique spatio-temporelle, sa « température », ses constituants sont très fortement liés à la durée de l'impulsion laser utilisée. Si celle-ci est dans un régime dit « court » de la microseconde à la nanoseconde, les effets principaux à l'origine du plasma sont des effets d'absorption linéaire avec des élévations de température locale de l'ordre de un à quelques degrés. C'est un processus qualifié de thermique. Il est considéré comme plus « grossier » sur la qualité du confinement du plasma dans un espace bien maîtrisé et de petite taille. Par contre, si la durée des impulsions est dans un régime dit « ultra-court » c'est à dire correspondant à des durées d'impulsions de l'ordre de quelques dizaines de picosecondes à la femtoseconde, alors les effets à l'origine du plasma seront une combinaison d'effets linéaires et non linéaires. D'ailleurs, plus la durée d'impulsion sera courte, plus les effets non linéaires seront favorisés. L'intérêt d'avoir recours à ces régimes réside dans l'accès à des processus dits « athermiques » permettant d'assurer un confinement plasma dans un espace très bien borné et de très petite taille sans élévation de température. Ce régime est donc plus favorable à la viabilité cellulaire à priori ainsi qu'à la haute résolution. Dans le cas de la présente invention, les principaux résultats ont été obtenus entre 5 et 10 picosecondes, un régime qui mixe à la fois les effets linéaires et non-linéaires. Ils ont permis de démontrer la capacité à imprimer sans couche sacrificielle à la fois des milieux homogènes et des milieux colloïdaux.
- La troisième étape correspond à la création de la bulle de cavitation (82) dans le milieu. Cette bulle est la résultante de la recombinaison des constituants du plasma en un gaz sous pression. La recombinaison est basée sur de nombreux processus physiques complexes comme des effets de champ, des recombinaisons radiatives et non radiatives, des effets tunnel, etc... La cavitation est très fortement dépendante de la taille et de la qualité du plasma initial (81). La cavitation (82) apparaît après environ une microseconde suite à l'absorption par le laser et à la création du plasma. Elle peut avoir une forme sphérique mais peut aussi avoir une forme allongée ou annulaire. Tout dépend du plasma initial et de sa forme. La polarisation du laser et la répartition géométrique de son énergie au plan focal influent directement sur la forme du plasma et donc sur la forme de la bulle de cavitation. Ainsi, pour obtenir des résultats plus reproductibles on privilégiera des formes isotropes, comme une polarisation laser circulaire.
- enfin la quatrième étape correspond à la phase dite d'hydrodynamique où la bulle de cavitation (82) va grandir, se déformer, provoquer des mouvements du liquide, etc... Les différents phases de ces phénomènes hydrodynamiques sont pour partie déjà connus au travers de certaines théories comme celles de Pearson ou Wortington.... La résultante finale est la création d'un jet de matière (83) au niveau de la surface libre du liquide. La tension de surface du liquide, la distance de la bulle à la surface libre, la viscosité du liquide font partie des paramètres les plus influents sur la forme et la dynamique de ce jet (83).

Ainsi, l'impression sans couche sacrificielle va être dépendante d'un très grand nombre de paramètres à la fois liés au laser et à l'encre utilisée. La maîtrise du film d'encre par l'équipement décrit selon l'invention est un moyen de réguler une partie des disparités possibles (sédimentation, séchage, épaisseur variable et non maîtrisée,...) lors de l'impression. En outre, les possibilités de modulation du débit et de l'épaisseur dudit film par les moyens de pression et d'aspiration, pourraient permettre de moduler la taille, la forme et la dynamique des jets. Ainsi, avec une telle invention, il devient envisageable de réduire le range des paramètres lasers nécessaires pour moduler les jets. L'impact direct d'un tel choix serait d'utiliser un laser beaucoup plus simple dans sa définition, plus stable et surtout beaucoup moins coûteux car moins versatile.

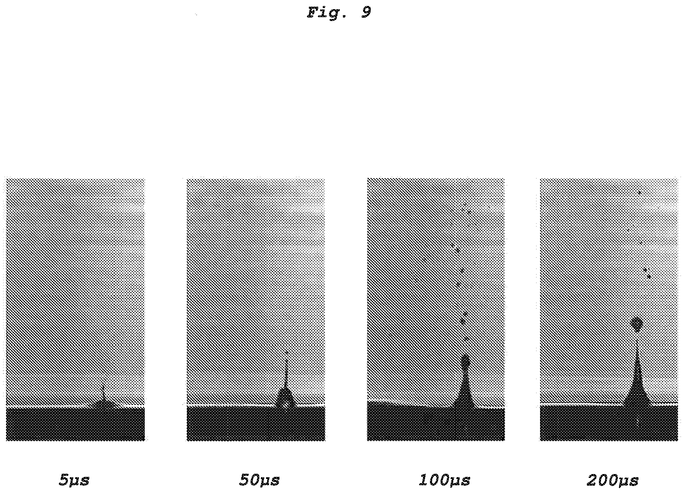

La figure 9 illustre des jets de matière réels, générés par laser sans couche sacrificielle. Les 4 photographies de cette figure, correspondent chacune à un temps spécifique après la focalisation du tir laser dans le film (4) d'encre. La première photo a été prise 5*µ*s après le tir, la seconde 50*µ*s après et ainsi de suite. Cette technique d'imagerie par ombroscopie s'appelle communément imagerie résolue en temps. Elle permet de décomposer des événements hyper-rapides par le biais de prise de photos grâce à des temps d'éclairage très courts. Cette série de photos permet d'illustrer le principe de la génération de jets par laser comme expliqué sur la figure 8 précédente. On peut voir aux temps courts la création d'un dôme de forme pyramidale surmonté d'un premier jet très fin puis aux temps longs on peut constater la montée d'un jet beaucoup plus imposant duquel se détache une ou plusieurs gouttes. Dépendant de la distance entre la surface libre du film (4) et le substrat receveur sur lequel on veut imprimer, ce sont une ou plusieurs de ces gouttelettes qui vont venir se déposer. Parfois, il peut arriver que la distance entre l'encre et le substrat d'impression soit suffisamment petite, en général moins de 500*µ*m, pour que le jet intercepte directement la surface du substrat receveur. On parle alors de régime de transfert. Dans tous les cas, que le mécanisme soit de dépôt de gouttelettes ou de transfert, on parlera d'impression par laser ver l'avant.

Les figures suivantes 10, 11, 12, 13 et 14 illustrent les résultats obtenus en impression sans couche sacrificielle. Ces résultats prouvent que les jeux de paramètres utilisés dans le cadre de cette invention:
- laser (régime picoseconde, la dizaine de microJoules, la longueur d'onde dans le proche infrarouge, la polarisation... )
- encre (viscosité, tension de surface, densité, épaisseur... )
- système (vitesse de balayage, motif utilisé, focalisation...)
permettent d'imprimer des objets homogènes comme inhomogènes, ce qui n'avait jamais été démontré jusque là.

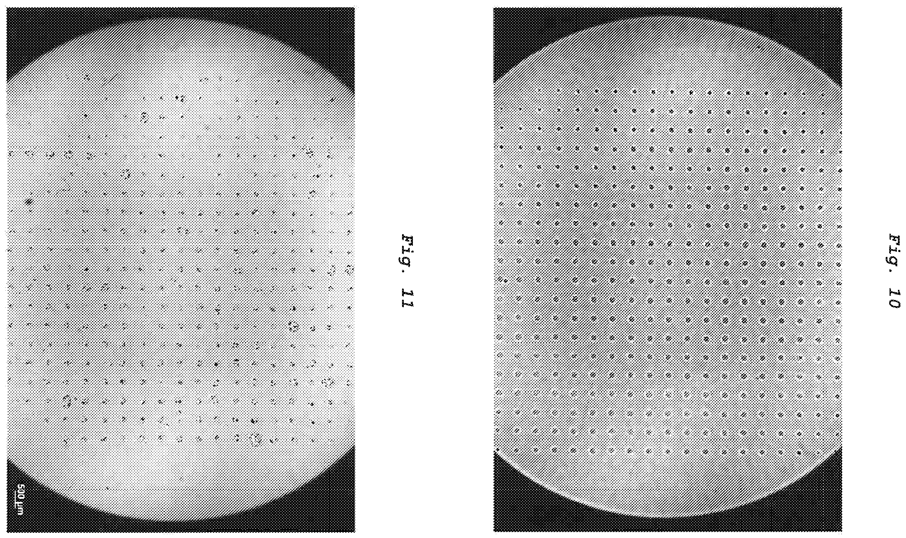

Ainsi, la figure 10 présente un résultat très reproductible d'impression laser sans couche sacrificielle d'une encre homogène constituée principalement d'eau. Chaque goutte imprimée apparaît comme un petit cercle sur l'image. Le grand cercle (séparant la zone grise de la zone noire) correspond tout simplement au champ imagé par le microscope utilisé pour prendre ce cliché. Les gouttes imprimées font typiquement 100*µ*m de diamètre et sont distantes de 500*µ*m les unes des autres. Ce résultat a été obtenu au sein de l'équipement selon l'invention en mode statique (sans rechargement continu).

La figure 11 présente un résultat homogène d'impression laser sans couche sacrificielle d'une encre colloïdale faite d'eau, de surfactant et de microbilles de 5*µ*m de diamètre chacune. Le résultat de l'impression montre la capacité de cette invention à déposer des gouttelettes enfermant un petit nombre de microbilles, en moyenne 2 à 3 par gouttelette. C'est la preuve que l'impression sans couche sacrificielle peut atteindre des performances de résolution très élevées sur des milieux colloïdaux (ce qui n'avait jamais été démontré jusque là). Ce résultat a été obtenu au sein de l'équipement selon l'invention en mode statique (sans rechargement continu).

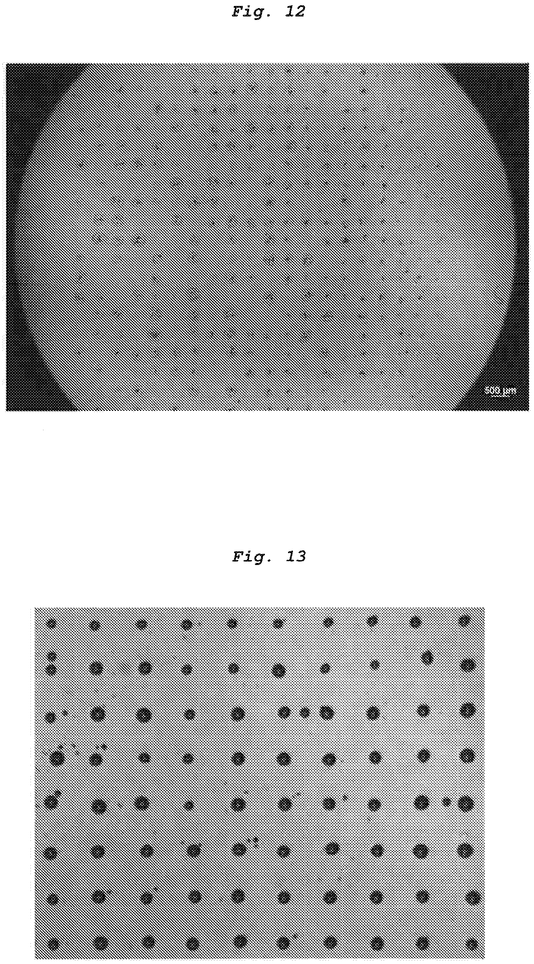

La figure 12 présente un résultat relativement homogène d'impression laser sans couche sacrificielle d'une encre cellulaire. Cette impression constitue une première comme pour les microbilles. Il est très probant et ouvre des champs d'utilisation de cette invention très larges. Les disparités de l'impression visibles sur l'image sont essentiellement liées aux disparités de l'encre utilisée et déposée sur la lame (3) qui a sédimentée et s'est agrégée par paquets. En effet, ce résultat a été obtenu en mode statique, c'est à dire sans faire fonctionner l'équipement selon l'invention dans un mode à rechargement continu dynamique. Le but de ce résultat était avant tout de prouver la capacité de l'impression sans or à imprimer des cellules vivantes. Là encore, ce résultat a été obtenu au sein de l'équipement selon l'invention en mode statique (sans rechargement continu).

La figure 13 présente un résultat d'impression relativement homogène d'impression laser sans couche sacrificielle d'une encre homogène obtenu avec l'équipement selon l'invention travaillant en mode dynamique, c'est à dire avec le système fluidique travaillant en rechargement continu.

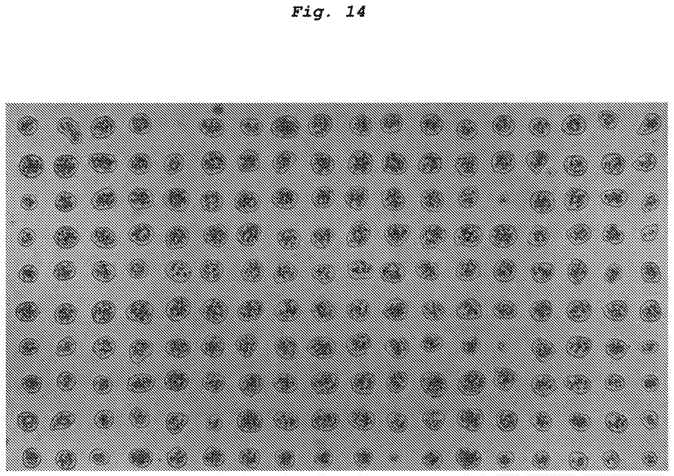

Enfin, la figure 14 est une autre illustration de la capacité de la technologie d'impression sans couche sacrificielle à pouvoir être optimisée en fonction des besoins. En effet, sur cette image, on peut voir un champ de gouttelettes imprimées dans les mêmes conditions que celles décrites jusqu'à présent à une seule différence près : la présence d'un agent absorbant incorporé à l'encre. Dans cet exemple, il s'agissait de mélanine, un composé biologique naturel ayant une très forte absorption à la longueur d'onde du laser utilisé pour ces expériences, à savoir 1030nm. Ainsi, l'ajout de ce composé a permis de travailler à des énergies laser moindres pour permettre l'absorption laser, puis la création du plasma et enfin l'hydrodynamique de la bulle de cavitation. Ce résultat a été obtenu au sein de l'équipement selon l'invention en mode statique (sans rechargement continu).

### Description d'une autre variante de réalisation

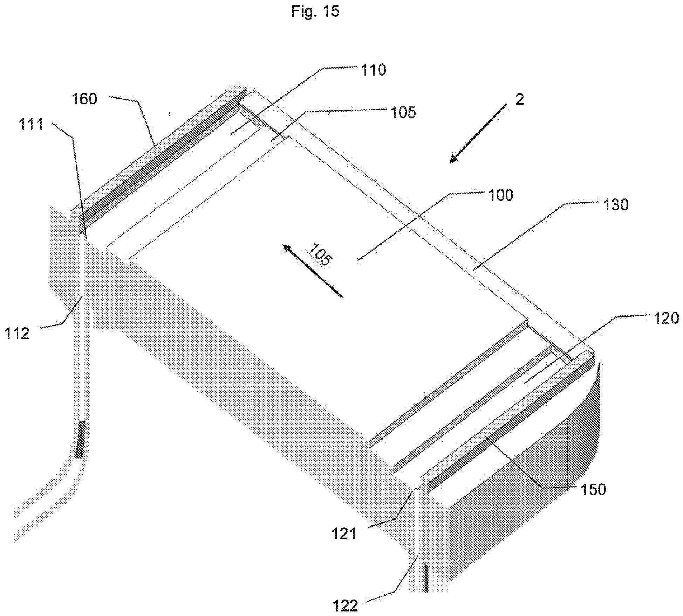

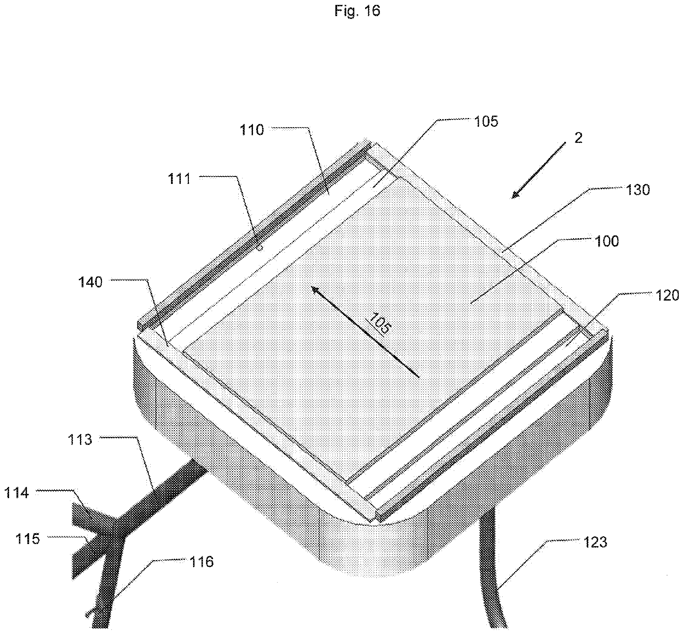

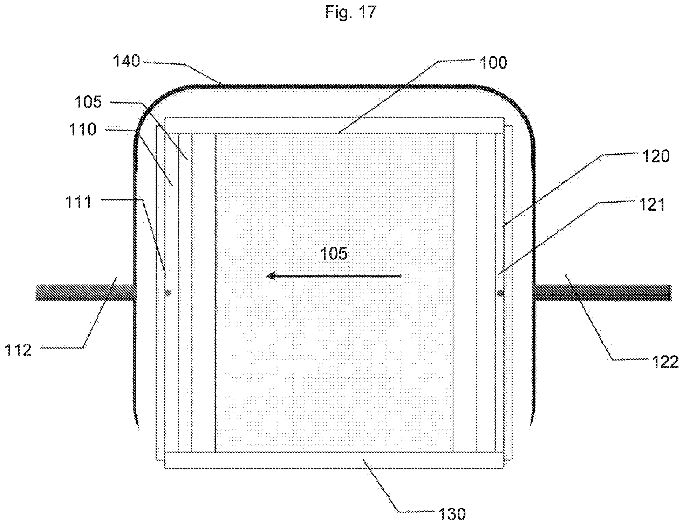

Les figures 15 à 17 représentent des vues respectivement en coupe partielle, en perspective et de dessus d'un autre exemple de lame (2), présentant une première surélévation (105) optionnelle, et un plateau (100) en forme de mésa, dont la surface supérieure est plane. Cette surface plane définit la zone d'interaction (100) entre le fluide et un faisceau d'excitation et/ou d'observation, par exemple un faisceau laser.

La lame présente, de part et d'autre de ce plateau (100), une cannelure transversale (110, 120).

Chacune des cannelures (110, 120) communique par un trou (111, 121) avec un conduit respectivement (112, 122) traversant verticalement la lame (2) et débouchant dans la cannelure correspondante respectivement (110, 120).

L'écoulement du fluide se produit selon une direction représentée par la flèche (105) correspondant à la direction longitudinale, entre une première cannelure transversale (110) et une seconde cannelure transversale (120).

La première cannelure (110) sert normalement à l'alimentation en fluide, qui traverse le plateau (100) avant de s'écouler dans la seconde cannelure (120) où le fluide est ensuite aspiré. Il est toutefois également possible de modifier le sens d'écoulement transitoirement, de façon à assurer un écoulement en sens alternés à la surface (100) du plateau.

Des tubes (113, 123) sont raccordés respectivement aux conduits (111, 121) pour l'alimentation et/ou l'aspiration de fluide portant les particules transférables. L'un des conduits peut être raccordé par une vanne multivoie à plusieurs arrivées (114 à 116) de fluides de natures différentes. Chacune de ces voies peut travailler soit en débit (du type pousse seringue) soit en pression

Les deux autres bords du plateau (100), non adjacents aux cannelures (110, 120) sont bordés optionnellement par un rebord (130, 140), pour former une aire délimité de circulation du fluide. De même, les bords latéraux extérieurs de la lame sont délimités par des rebords (150, 160).

Le pilotage du débit d'alimentation et/ou d'aspiration permet de contrôler l'écoulement pour assurer une répartition homogène du liquide sur la partie ouverte de la tête d'impression.

Une première solution consiste à prévoir une bouche d'entrée ainsi qu'une sortie rectangulaire, les bouches d'entrée et de sortie étant définies par la surface supérieure des cannelures transversales (110, 120) comme indiqué sur les figures 15 à 17.

Les rebords latéraux (130, 140) assurent que la forme du ménisque liquide dans le plan contenant l'axe (105) puisse être contrôlé. Ils peuvent être physiques ou chimiques.

Selon un mode de réalisation particulier de l'invention, le film liquide présente une épaisseur parfaitement plate ou plus fine sur les bords afin que l'écoulement ait une résistance hydrodynamique plus élevée sur les bords.

Cette solution peut aussi comporter :
- différents éléments pour pouvoir contrôler l'épaisseur du liquide
- des murs (150, 160) en amont et en aval sur lesquels l'encrage de ligne de contact est facilité
- une plateforme centrale (100) de surélévation pour réduire l'épaisseur du film dans la zone d'interaction.

Une autre solution pour alterner la nature du fluide consiste à alterner un fluide contenant des particules transférables, avec un liquide inerte.

Une autre solution comporte un tuyau d'évacuation de liquide avec au moins deux voies.

Le fonctionnement est alors le suivant :
Etape 1 d'initialisation : Afin de créer un pont liquide sur la tête d'impression reliant le point d'injection et le point d'évacuation un process de contrôle est mis en place.

Une première solution consiste à injecter du liquide depuis les cartouches vers la tête depuis le point d'injection et d'évacuation.

Une autre solution consiste à injecter du liquide depuis la cartouche vers la tête uniquement depuis le point d'injection.

Une autre solution consiste à utiliser une texture permettant le mouillage total de la partie ouverte de la tête d'impression.

Etape 2 d'automatisation : Une fois le pont liquide créé, l'épaisseur du film est contrôlée en retirant du liquide soit depuis le point d'injection soit depuis le point d'évacuation.

Un premier exemple de réalisation établit un flux continu et contrôle l'épaisseur du film en imposant un débit entre le point d'injection et le point d'évacuation.

Un autre exemple de réalisation établit un flux continu et contrôle l'épaisseur du film en imposant une différence de pression entre le point d'injection et le point d'évacuation.

Un autre exemple de réalisation opère en mode discontinu :
- injection d'un volume imposé depuis le point d'injection
- Puis réglage de l'épaisseur désirée en retirant du liquide depuis le point d'injection ou le point d'évacuation.

Cette opération est répétée de façon cyclique.

Afin de mieux contrôler le film liquide sur la tête d'impression, des senseurs peuvent être intégrés.

Un exemple de réalisation utilise un système confocal pour mesurer l'épaisseur du film. Un autre exemple de réalisation utilise un système de détection optique sur les tubes d'injection et d'évacuation. Un développement avantageux permet de détecter le passage de bulles, particulièrement sur la voie d'évacuation. Un développement avantageux permet de détecter des concentrations, particulièrement sur la voie d'injection.

## Revendications

1. Equipement d'impression additive comportant un moyen d'excitation énergétique orientable pour produire une interaction ponctuelle avec un fluide recouvrant une lame (2), afin de provoquer un jet orienté en direction d'une cible (11) (11), ledit fluide étant constitué par un vecteur liquide contenant des inhomogénéités transférables, **caractérisé en ce que**
• ledit fluide forme un film (4) liquide d'une épaisseur inférieure à 500 *µ*m,
• sur une lame (2) présentant au moins une zone (3) permettant l'interaction avec le laser dans laquelle débouche au moins une entrée (7), ladite zone d'interaction (3) débouchant dans au moins une sortie (9)
• l'équipement comportant en outre des moyens de circulation du fluide entre ladite entrée (7) et ladite sortie (9).

2. Equipement d'impression additive selon la revendication 1 **caractérisé en ce que** l'épaisseur dudit film (4) est comprise entre 20 et 100 *µ*m.

3. Equipement d'impression additive selon la revendication 1 **caractérisé en ce que** l'épaisseur dudit film (4) est comprise entre 3 et 10 fois la taille nominale desdites particules transférables.

4. Equipement d'impression additive selon la revendication 1 **caractérisé en ce que** la surface de ladite zone d'interaction (3) est supérieure 0,05 mm².

5. Equipement d'impression additive selon la revendication 1 **caractérisé en ce que** ladite entrée (7) débouche dans une partie latérale de ladite zone d'interaction (3).

6. Equipement d'impression additive selon la revendication 1 **caractérisé en ce que** ladite zone d'interaction (3) présente une partie périphérique débouchant latéralement dans ladite sortie (9).

7. Equipement d'impression additive selon la revendication 1 **caractérisé en ce que** ladite entrée (7) et ladite sortie (9) sont constituées par des canaux tubulaires raccordés à la zone de raccordement (3).

8. Equipement d'impression additive selon la revendication 1 **caractérisé en ce que** lesdits moyens de circulation du fluide entre ladite entrée (7) et ladite sortie (9) comprennent des moyens de pilotage du débit d'injection (ou de la pression positive exercée sur le fluide) et du débit d'aspiration pour contrôler le débit du fluide (ou de la pression négative exercée sur le fluide) dans la zone d'interaction.

9. Equipement d'impression additive selon la revendication précédente **caractérisé en ce que** lesdits moyens de pilotage du débit d'injection et du débit d'aspiration sont asservis à la mesure de l'épaisseur du film (4), pour contrôler l'épaisseur du film (4) dans la zone d'interaction (3).

10. Equipement d'impression additive selon la revendication 1 **caractérisé en ce qu'**il comporte une pluralité de zones d'interaction comportant chacune une entrée (7) et une sortie (9).

11. Equipement d'impression additive selon la revendication 1 **caractérisé en ce que** ledit moyen d'excitation énergétique est constitué par un laser.

12. Equipement d'impression additive selon la revendication précédente **caractérisé en ce que** la zone d'interaction est transparente dans la bande de longueur d'onde du laser et de l'imagerie et ne présente pas de couche sacrificielle.

13. Equipement d'impression additive selon la revendication précédente **caractérisé en ce que** ledit fluide est chargé d'un pigment absorbant dans la longueur d'onde d'émission du laser.

14. Equipement d'impression additive selon la revendication 11 **caractérisé en ce qu'**il comporte des moyens d'imagerie de la zone d'interaction pour le pilotage du laser en fonction de la densité de inhomogénéités.

15. Equipement d'impression additive selon la revendication 1 **caractérisé en ce que** ledit moyen d'excitation énergétique est constitué par un générateur d'ondes acoustiques.

16. Equipement d'impression additive selon la revendication 1 **caractérisé en ce que** ladite lame (2), présente un plateau (100) en forme de mésa, dont la surface supérieure définit la zone d'interaction (100) entre le fluide et un faisceau d'excitation et/ou d'observation, ladite lame (2) présentant de part et d'autre de ce plateau (100), une cannelure transversale (110, 120), chacune desdites cannelures (110, 120) communiquant par un trou (111, 121) avec un conduit respectivement (112, 122) traversant verticalement la lame (2) et débouchant dans la cannelure correspondante respectivement (110, 120).

17. Procédé d'impression additive par un équipement comportant un moyen d'excitation énergétique orientable pour produire une interaction ponctuelle avec un fluide recouvrant une lame (2), afin de provoquer un jet orienté en direction d'une cible (11), ledit fluide étant constitué par un vecteur liquide contenant des inhomogénéités transférables, **caractérisé en ce que** ledit fluide forme un film (4) liquide d'une épaisseur inférieure à 500 *µ*m circulant entre un conduit d'entrée (7) et un conduit de sortie (9) d'une lame (2) présentant au moins une zone permettant l'interaction avec le laser, et dans laquelle débouche au moins une entrée (7).

18. Procédé d'impression additive selon la revendication précédente **caractérisé en ce que** le niveau d'énergie par impulsion est commandé par un calculateur en fonction du résultat de mesure de caractéristiques du fluide présent dans la zone d'interaction, lesdites mesures comprenant la densité en particules, et/ou la viscosité, et/ou l'épaisseur du film (4).

## Patentansprüche

1. Additivdruckeinrichtung, die ein ausrichtbares Mittel zur energetischen Erregung zum Erzeugen einer punktuellen Wechselwirkung mit einem Fluid umfasst, das einen Träger (2) überzieht, um einen in Richtung eines Ziels (11) (11) ausgerichteten Strahl zu erzeugen, wobei das Fluid aus einem flüssigen Vektor besteht, der übertragbare Inhomogenitäten enthält, **dadurch gekennzeichnet, dass**
• das Fluid einen Flüssigkeitsfilm (4) mit einer Dicke von weniger als 500 µm bildet,
• auf einem Träger (2), der wenigstens einen Bereich (3) aufweist, der eine Wechselwirkung mit dem Laser ermöglicht, in den wenigstens ein Einlass (7) mündet, wobei der Wechselwirkungsbereich (3) in wenigstens einen Auslass (9) mündet,
• die Einrichtung ferner Mittel zum Zirkulieren des Fluids zwischen dem Einlass (7) und dem Auslass (9) umfasst.

2. Additivdruckeinrichtung nach Anspruch 1, **dadurch gekennzeichnet, dass** die Dicke des Films (4) zwischen 20 und 100 µm beträgt.

3. Additivdruckeinrichtung nach Anspruch 1, **dadurch gekennzeichnet, dass** die Dicke des Films (4) zwischen dem 3- bis 10-fachen der Nenngröße der übertragbaren Partikel beträgt.

4. Additivdruckeinrichtung nach Anspruch 1, **dadurch gekennzeichnet, dass** die Oberfläche des Wechselwirkungsbereichs (3) größer als 0,05 mm² ist.

5. Additivdruckeinrichtung nach Anspruch 1, **dadurch gekennzeichnet, dass** der Einlass (7) in einen seitlichen Teil des Wechselwirkungsbereichs (3) mündet.

6. Additivdruckeinrichtung nach Anspruch 1, **dadurch gekennzeichnet, dass** der Wechselwirkungsbereich (3) einen peripheren Teil aufweist, der seitlich in den Auslass (9) mündet.

7. Additivdruckeinrichtung nach Anspruch 1, **dadurch gekennzeichnet, dass** der Einlass (7) und der Auslass (9) aus rohrförmigen Kanälen bestehen, die an den Anschlussbereich (3) angeschlossen sind.

8. Additivdruckeinrichtung nach Anspruch 1, **dadurch gekennzeichnet, dass** die Fluidumwälzmittel zwischen dem Einlass (7) und dem Auslass (9) Mittel zum Steuern des Einspritzdurchsatzes (oder des auf das Fluid ausgeübten Überdrucks) und des Absaugungsdurchsatzes (oder des auf das Fluid ausgeübten Unterdrucks) enthalten, um den Fluiddurchsatz im Wechselwirkungsbereich zu regeln.

9. Additivdruckeinrichtung nach dem vorhergehenden Anspruch, **dadurch gekennzeichnet, dass** die Mittel zur Steuerung des Einspritzdurchsatzes und den Ansaugdurchsatzes von der Messung der Dicke des Films (4) abhängen, um die Dicke des Films (4) in dem Wechselwirkungsbereich (3) zu regeln.

10. Additivdruckeinrichtung nach Anspruch 1, **dadurch gekennzeichnet, dass** sie eine Vielzahl von Wechselwirkungsbereichen umfasst, von denen jeder einen Einlass (7) und einen Auslass (9) umfasst.

11. Additivdruckeinrichtung nach Anspruch 1, **dadurch gekennzeichnet, dass** das Mittel zur energetischen Erregung aus einem Laser besteht.

12. Additivdruckeinrichtung nach dem vorhergehenden Anspruch, **dadurch gekennzeichnet, dass** der Wechselwirkungsbereich in dem Wellenlängenband des Lasers und der Bildgebung transparent ist und keine Opferschicht aufweist.

13. Additivdruckeinrichtung nach dem vorhergehenden Anspruch, **dadurch gekennzeichnet, dass** das Fluid mit einem Pigment beladen ist, das in der Emissionswellenlänge des Lasers absorbierend ist.

14. Additivdruckeinrichtung nach Anspruch 11, **dadurch gekennzeichnet, dass** sie Bildgebungsmittel für den Wechselwirkungsbereich zum Steuern des Lasers in Abhängigkeit von der Dichte der Inhomogenitäten umfasst.

15. Additivdruckeinrichtung nach Anspruch 1, **dadurch gekennzeichnet, dass** das Mittel zur energetischen Erregung aus einem Schallwellengenerator besteht.

16. Additivdruckeinrichtung nach Anspruch 1, **dadurch gekennzeichnet, dass** der Träger (2) eine Platte (100) in Mesaform aufweist, deren obere Oberfläche den Wechselwirkungsbereich (100) zwischen dem Fluid und einem Erregungs- und/oder Beobachtungsstrahl definiert, wobei der Träger (2) auf beiden Seiten dieser Platte (100) eine Querrille (110, 120) aufweist, wobei jede der Rillen (110, 120) durch ein Loch (111, 121) mit jeweils einer Leitung (112, 122) kommuniziert, die vertikal durch den Träger (2) läuft und jeweils in die entsprechende Rille (110, 120) mündet.

17. Additivdruckverfahren durch eine Einrichtung, die ein ausrichtbares Mittel zur energetischen Erregung zum Erzeugen einer punktuellen Wechselwirkung mit einem Fluid umfasst, das einen Träger (2) überzieht, um einen in Richtung eines Ziels (11) ausgerichteten Strahl zu erzeugen, wobei das Fluid aus einem flüssigen Vektor besteht, der übertragbare Inhomogenitäten enthält, **dadurch gekennzeichnet, dass** das Fluid einen Flüssigkeitsfilm (4) mit einer Dicke von weniger als 500 µm bildet, das zwischen einer Leitung des Einlasses (7) und einer Leitung des Auslasses (9) eines Trägers (2) zirkuliert, der wenigstens einen Bereich aufweist, der die Wechselwirkung mit dem Laser ermöglicht und in den wenigstens ein Einlass (7) mündet.

18. Additivdruckverfahren nach dem vorhergehenden Anspruch, **dadurch gekennzeichnet, dass** das Energieniveau pro Impuls durch einen Rechner in Abhängigkeit von dem Ergebnis der Messung von Eigenschaften des in dem Wechselwirkungsbereich vorhandenen Fluids vorgegeben wird, wobei die Messungen die Partikeldichte und/oder die Viskosität und/oder die Dicke des Films (4) enthalten.

## Claims

1. Additive printing apparatus comprising a directable energetic excitation means for producing point interaction with a fluid covering a slide (2), in order to cause a jet directed toward a target (11) (11), said fluid consisting of a liquid vector containing transferable inhomogeneities, **characterized in that**
• said fluid forms a liquid film (4) having a thickness of less than 500 µm,
• on a slide (2) having at least one region (3) for interaction with the laser, into which region at least one inlet (7) leads, said interaction region (3) leading into at least one outlet (9),
• the apparatus further comprising means for circulating the fluid between said inlet (7) and said outlet (9).

2. Additive printing apparatus according to claim 1, **characterized in that** the thickness of said film (4) is between 20 and 100 µm.

3. Additive printing apparatus according to claim 1, **characterized in that** the thickness of said film (4) is between 3 and 10 times the nominal size of said transferable particles.

4. Additive printing apparatus according to claim 1, **characterized in that** the surface of said interaction region (3) is greater than 0.05 mm².

5. Additive printing apparatus according to claim 1 **characterized in that** said inlet (7) leads into a lateral part of said interaction region (3).

6. Additive printing apparatus according to claim 1, **characterized in that** said interaction region (3) has a peripheral part leading laterally into said outlet (9).

7. Additive printing apparatus according to claim 1, **characterized in that** said inlet (7) and said outlet (9) consist of tubular channels connected to the connection region (3).

8. Additive printing apparatus according to claim 1, **characterized in that** said means for circulating the fluid between said inlet (7) and said outlet (9) comprise means for controlling the injection flow rate (or the positive pressure exerted on the fluid) and suction flow rate (or negative pressure exerted on the fluid) in order to control the flow rate of the fluid in the interaction region.

9. Additive printing apparatus according to the preceding claim, **characterized in that** said means for controlling the injection flow rate and the suction flow rate are dependent on the measurement of the thickness of the film (4), in order to control the thickness of the film (4) in the interaction region (3).

10. Additive printing apparatus according to claim 1, **characterized in that** said apparatus comprises a plurality of interaction regions each comprising an inlet (7) and an outlet (9).

11. Additive printing apparatus according to claim 1, **characterized in that** said energetic excitation means consists of a laser.

12. Additive printing apparatus according to the preceding claim, **characterized in that** the interaction region is transparent in the wavelength band of the laser and in the imaging wavelength band and does not have a sacrificial layer.

13. Additive printing apparatus according to the preceding claim, **characterized in that** said fluid is loaded with a pigment which is absorbent in the emission wavelength of the laser.

14. Additive printing apparatus according to claim 11, **characterized in that** said apparatus comprises means for imaging the interaction region in order to control the laser according to the density of inhomogeneities.

15. Additive printing apparatus according to claim 1, **characterized in that** said energetic excitation means consists of an acoustic wave generator.

16. Additive printing apparatus according to claim 1, **characterized in that** said slide (2) has a mesa-shaped plate (100), the upper surface of which defines the region (100) of interaction between the fluid and an excitation and/or observation beam, said slide (2) having, on either side of this plate (100), a transverse groove (110, 120), each of said grooves (110, 120) communicating, by a hole (111, 121), with a particular duct (112, 122), that passes vertically through the slide (2) and leads into the corresponding groove (110, 120).

17. Additive printing method using an apparatus comprising a directable energetic excitation means for producing point interaction with a fluid covering a slide (2), in order to cause a jet directed toward a target (11), said fluid consisting of a liquid vector containing transferable inhomogeneities, **characterized in that** said fluid forms a liquid film (4) having a thickness less than 500 µm and circulating between an inlet duct (7) and an outlet duct (9) of a slide (2) which has at least one region for interaction with the laser, and into which at least one inlet (7) leads.

18. Additive printing method according to the preceding claim, **characterized in that** the level of energy per pulse is controlled by a computer according to the result of measurement of characteristics of the fluid present in the interaction region, said measurements including the particle density, and/or the viscosity, and/or the thickness of the film (4).
